(19) Europäisches Patentamt
European Patent Office
Office européen des brevets

(11) Publication number: **0 148 366**
**A2**

(12) # EUROPEAN PATENT APPLICATION

(21) Application number: **84113566.8**

(22) Date of filing: **30.12.81**

(51) Int. Cl.⁴: **C 07 C 63/68**, C 07 C 51/363

(30) Priority: **29.12.80 US 220672**
**29.12.80 US 220674**
**26.01.81 US 228656**
**29.12.80 US 220664**
**29.12.80 US 220673**

(43) Date of publication of application: **17.07.85**
**Bulletin 85/29**

(84) Designated Contracting States: **BE DE FR GB IT NL**

(60) Publication number of the earlier application in accordance with Art. 76 EPC: **0055630**

(71) Applicant: **OCCIDENTAL CHEMICAL CORPORATION, P.O. Box 189, Niagara Falls New York 14302 (US)**

(72) Inventor: **Fifolt, Michael J., 2225 Staley Road, Grand Island New York 14072 (US)**
Inventor: **Foster, Arthur M., 32870 Robin Hood, Birmingham Michigan 48010 (US)**
Inventor: **Cotter, Byron R., 262 Laurie Lane, Grand Island New York 14072 (US)**

(74) Representative: **Myerscough, Philip Boyd et al, J.A.Kemp & Co. 14, South Square Gray's Inn, London, WC1R 5EU (GB)**

(54) **4,5-difluorophthaloyl fluoride and its preparation.**

(57) 4,5-Difluorophthaloyl fluoride, a novel compound of use as an intermediate in the preparation of fluorine-containing aromatic compounds, is prepared by reacting 4,5-dichlorophthalic anhydride with anhydrous potassium fluoride.

**EP 0 148 366 A2**

"4,5-DIFLUOROPHTHALOYL FLUORIDE AND ITS PREPARATION"

This invention relates to 4,5-difluorophthaloyl fluoride and to a process for its preparation. The compound of the invention is useful as a chemical intermediate for the preparation of a wide variety of end products including pharmaceuticals and agricultural chemicals such as pesticides, and, in particular, as a reactant in novel and advantageous methods for the synthesis of fluoroanthranilic acids, fluoroanilines, fluorobenzoic acids and other fluorinated aromatic compounds.

In EP-A-55630, from which this Application is divided we describe and claim fluorophthalamic compounds of the formula

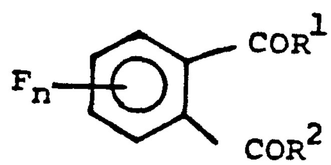

wherein n is 1 or 2, and $R^1$ is $-NH_2$ and $R^2$ is -OH or $-ONH_4$ or $R^1$ and $R^2$ together are >NH or >NM, wherein M is an alkali metal.

The fluorophthalamic compounds are produced through the reaction sequence:

(A) chlorophthalic anhydrides of the formula

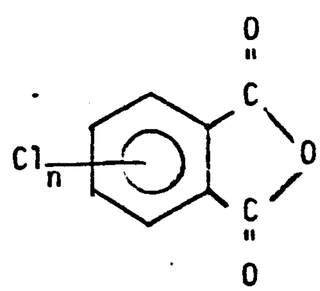

wherein n is 1 or 2, are reacted with potassium fluoride or cesium fluoride to form fluorophthalic anhydrides of the formula

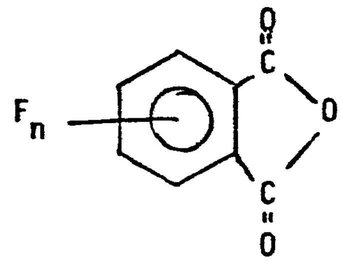

wherein n is 1 or 2;

(B) the fluorophthalic anhydride of (A) is then reacted with ammonia to form an ammonium fluorophthalamate;

(C) the ammonium fluorophthalamate of (B) is acidified to form a fluorophthalamic acid;

(D) the fluorophthalamic acid of (C) is dehydrated to form a fluorophthalimide; and

(E) the fluorophthalimide of (D) is reacted with an alkali metal base to form an alkali metal fluorophthalimide.

The fluorination step (A) of the process, that is the preparation of fluorophthalic anhydride by reaction of chlorophthalic anhydride with potassium fluoride or cesium fluoride, may be carried out under a wide range of conditions. The temperature of the reaction may vary considerably for example, from 75° or lower to 300°C or higher, depending, in part on the particular chlorophthalic anhydride reactant used and whether or not a catalyst is employed. The reaction is preferably carried out in the presence of a catalyst, at a temperature of 80° to 280°C. When potassium fluoride is employed as the fluorinating agent, it is preferred to carry out the reaction at higher temperatures, such as 180° to 275°C. When cesium fluoride is employed as the fluorinating agent, lower temperatures, such as 80° to 200°C are preferred.

The preferred catalysts that may be employed in the fluorination steps are polyether catalysts, such as crown ethers, polyethylene glycols or alkoxy polyethylene glycols, such as polyethers typically having a molecular weight ranging from 200 to 25,000. Typically, the catalysts are

employed in amounts of 0.1 to 20 percent, and preferably 5.0 to 15 percent by weight, based on the amount of chlorophthalic anhydride.

The fluorination reaction is preferably and conveniently carried out under atmospheric pressure conditions. However, super-atmospheric and sub-atmospheric conditions may be employed, if desired.

It is preferred, but not essential, to employ a catalyst when potassium fluoride is used as the fluorinating agent. When cesium fluoride is used, a catalyst may be employed, but is not preferred, due to the higher reactivity of that fluorinating agent.

Although it is preferred to run the fluorination reaction neat, an inert solvent, such as dipolar aprotic solvent may be employed if desired. Suitable solvents include, for example, dimethylsulfoxide; dimethylformamide, N-methyl-2-pyrrolidone; sulfolane and hexamethylphosphoramide.

The proportion of reactants for the fluorination step may vary widely. Generally it is preferred to employ 20 to 25 percent molar excess of potassium fluoride or cesium fluoride fluorinating agent.

If anhydrous potassium fluoride is utilized as the fluorination agent with 4,5-dichlorophthalic anhydride as a reactant and the molar ratio of potassium fluoride: dichlorophthalic anhydride maintained at from 4:1 to 20:1 the novel compound 4,4-difluorophthaloyl fluoride can be produced in economic quantities along with the fluorophthalic anhydride of the disclosed reaction sequence. 4,5-Difluorophthaloyl fluoride is useful as a chemical intermediate in the preparation of various chemical end products, such as 4,5-difluorophthalimide and resins such as polyamides. Polyamides having fluorine substituents on the polymer chain may be prepared by polycondensation of 4,5-difluorophthaloyl fluoride with a suitable diamine, such as piperazine,

utilizing such known techniques as interfacial polymerization or solution polymerization.

4,5-Difluorophthalimide may be converted to its salts, especially the alkali metal salts, by reaction with a base, such as alkali metal base, such as potassium hydroxide. 4,5-Difluorophthalimide and salts thereof have been found useful as intermediates in the preparation of various fluorinated aromatic compounds, especially in the preparation of fluoroanthranilic acids as described in EP-A-55630.

The following Example is provided to further illustrate this invention and the manner in which it may be carried out. In the Example, all parts are by weight.

EXAMPLE

A reaction mixture was prepared by mixing and grinding together 21.7 parts of 4,5-dichlorophthalic anhydride and 58 parts of anhydrous potassium fluoride. The mixture was charged to a reaction vessel together with 2 parts of Carbowax ® MPEG 2000 catalyst, heated to about 210°C and maintained thereat for about 6.5 hours. The mixture was then cooled to about 25°C and allowed to stand for about 16 hours, then reheated and maintained at about 210°C for 7.5 hours. The reaction product was purified by vacuum distillation to yield 2.43 parts of product. Analysis of the product by $C^{13}$ nuclear magnetic resonance and infra-red analysis confirmed it to be 4,5-difluorophthaloyl fluoride.

## CLAIMS

1. 4,5-Difluorophthaloyl fluoride.

2. A process for the preparation of 4,5-difluorophthaloyl fluoride which comprises reacting 4,5-dichlorophthalic anhydride with anhydrous potassium fluoride.

3. A process according to claim 2 wherein the reaction is carried out in the absence of solvent.

4. A process according to claim 2 or 3 wherein the molar ratio of potassium fluoride to 4,5-dichlorophthalic anhydride is from 4:1 to 20:1.

5. A process according to claim 2, 3 or 4 wherein the reaction temperature is from 180° to 275°C.